# EUROPÄISCHE PATENTSCHRIFT

(11) **EP 0 278 556 B1**
(45) Veröffentlichungstag und Bekanntmachung des Hinweises auf die Patenterteilung: **14.04.1993**
(21) Anmeldenummer: 88200163.9
(22) Anmeldetag: 01.02.1988
(51) Int. Cl.: C12N 11/00, C12N 11/14, C12N 11/04

(54) **Kugelförmiger Biokatalysator und Verfahren zu seiner Herstellung**
Spherical biocatalyst and process for its preparation
Biocatalyseur en forme de bille et son procédé de préparation

(30) Priorität: 13.02.1987 DE 3704478
(43) Veröffentlichungstag der Anmeldung: 17.08.1988
(73) Patentinhaber: METALLGESELLSCHAFT AG, 60015 Frankfurt (DE)
(72) Erfinder: Sander, Ulrich, Dr., D-6382 Friedrichsdorf (DE)

(56) Entgegenhaltungen:
- AU-A- 482 149
- DE-A- 2 727 143
- FR-A- 2 422 699
- CHEMICAL ABSTRACTS, Band 79, Nr. 17, 29. Oktober 1973, Seite 172, Zusammenfassung Nr. 102068b, Columbus, Ohio, US; J.F. KENNEDY et al.: "Active, water-insoluble derivatives of D-glucose oxidase and alginic acid, chitin, and Celite", & CARBOHYD. RES. 1973, 28(1), 89-92
- CHEMICAL ABSTRACTS, Band 96, Nr. 25, 21. Juni 1982, Seite 581, Zusammenfassung Nr. 215965m, Columbus, Ohio, US; S.M.M. DIAS et al.: "Immobilization of yeasts on titanium activated inorganic supports", & BIOTECHNOL. LETT. 1982, 4(3), 203-8

## Beschreibung

Die Erfindung bezieht sich auf einen kugelförmigen Biokatalysator, der aus immobilisiertem biologisch aktivem Material sowie einer ionotropen gelartigen Matrix besteht, sowie auf ein Verfahren zu seiner Herstellung.

Aus der Veröffentlichung von Vorlop und Klein in Enzyme Technology, III. Rotenburg Fermentation Symposium 1982, 22.-24.09.1982, Springer-Verlag, 1983, Seiten 219-235, sind Biokatalysatoren der eingangs genannten Art bekannt. Beispielsweise ist in der Veröffentlichung gesagt, daß Biokatalysatoren mit einem Durchmesser von 0,1 bis 4 mm und guten mechanischen Eigenschaften hergestellt werden können, die aus einer vernetzten Alginat-Matrix und Mikroorganismen (Hefen, Bakterien) bestehen. Zur Herstellung der bekannten Biokatalysatoren werden die Alginate in Wasser gelöst, anschließend wird die wässrige Lösung in einem Autoklaven bei erhöhter Temperatur behandelt, danach werden Mikroorganismen in der Alginat-Lösung dispergiert, dann wird die Dispersion in eine Calciumchlorid-Lösung getropft, wobei die Tropfen in dieser Lösung zur Aushärtung 30 bis 60 Minuten verbleiben, und schließlich wird der Biokatalysator abgetrennt sowie gewaschen. Aus der Veröffentlichung ist ferner bekannt, daß auch die Biopolymere Pectin, Carragenan und Chitosan als Matrix verwendet werden können. Anstelle der Mikroorganismen können auch Zellen und Enzyme als biologisch aktives Material in der Matrix immobilisiert werden.

Es hat sich gezeigt, daß die bekannten Biokatalysatoren im Fermentationsreaktor aufschwimmen und im oberen Teil des Reaktors eine zusammenhängende Schicht bilden, da die Dichte der Katalysatorperlen etwa der Dichte der Lösung entspricht, die sich im Fermentationsreaktor befindet und deren Inhaltstoffe unter Mitwirkung des Katalysators zur Reaktion gebracht werden sollen. Das Aufschwimmen der bekannten Biokatalysatoren wirkt sich besonders nachteilig aus, wenn die Katalysatoren in einem Fermentationsreaktor eingesetzt werden, der nach dem Verfahrensprinzip des Fließbetts bzw. des Wirbelbetts betrieben wird. Der Erfinding liegt daher die Aufgabe zugrunde, einen Biokatalysator zu schaffen, der eine höhere Dichte als Wasser hat und einen störungsfreien Betrieb eines im Fließbett ablaufenden Fermentationsprozesses gewährleistet. Außerdem muß der Biokatalysator eine hohe Abriebfestigkeit besitzen und unter sterilen Bedingungen herstellbar sein. Ferner liegt der Erfindung die Aufgabe zugrunde, ein Verfahren zur Herstellung des Biokatalysators vorzuschlagen.

Die der Erfindung zugrundeliegende Aufgabe wird in überraschender Weise dadurch gelöst, daß die Matrix TiO₂ enthält, welches einen Teilchendurchmesser von 0,1 bis 1 µm hat. Es hat sich gezeigt, daß der Katalysator auch im Dauerbetrieb abriebfest ist und im feuchten Zustand ein spezifisches Gewicht von 1,1 bis 1,4 g/ml hat, wenn er einen TiO₂-Gehalt von 5 bis 30 Gew.% aufweist. Das TiO₂ hat eine große spezifische Oberfläche sowie einen hohen Anteil an ionenaktiven Bindungsvalenzen.

Der erfindungsgemäße Biokatalysator hat besonders vorteilhafte Eigenschaften, wenn die TiO₂-Teilchen an der Oberfläche mit Al₂O₃ und/oder AlO(OH) dotiert sind. Die Dotierung der TiO₂-Teilchen erfolgt in der Weise, daß sie mit einer 0,1- bis 2-molaren Aluminiumsalzlösung besprüht und dann bei 150 bis 500°C behandelt werden. Pro kg TiO₂ werden 10 bis 50 g der Aluminiumsalze, berechnet als Al₂O₃, verwendet. Bei der Temperaturbehandlung entsteht durch Hydrolyse des Aluminiumsalzes Al₂O₃ und/oder AlO(OH), das an der Oberfläche der TiO₂-Teilchen fest verankert ist. Als Aluminiumsalz kommt insbesondere AlCl₃ zur Anwendung. Es hat sich gezeigt, daß insbesondere die mit Al₂O₃ bzw. AlO(OH) dotierten TiO₂-Teilchen an der Oberfläche freie Valenzen haben, die sich an die Makromoleküle der Matrix anlagern, wodurch eine sehr stabile Suspension ausgebildet wird.

In weiterer Ausgestaltung der Erfindung ist vorgesehen, daß als biologisch aktives Material Enzyme, Mikroorganismen oder Zellen verwendet werden und daß die Matrix aus Biopolymeren, wie Alginat, Pectin, Carragenan oder Chitosan, besteht. Es wurde festgestellt, daß der erfindungsgemäße Biokatalysator bei Verwendung der vorgenannten Substanzen gute mechanische Eigenschaften, eine hinreichend große Dichte sowie eine gute Aktivität besitzt. Nach der Erfindung ist vorgesehen, daß der Biokatalysatoren einen Teilchendurchmesser von 0,05 bis 5 mm hat. Bei Fermentationsreaktionen mit Gasbildung oder Gaszufuhr werden vorteilhaft Katalysatorteilchen verwendet, die einen Durchmesser von 0,5 bis 5 mm haben, während bei Fermentationsreaktionen ohne Gasbildung oder Gaszufuhr vorzugsweise Katalysatorteilchen mit einem Durchmesser von 0,05 bis 0,5 mm zum Einsatz kommen.

Die der Erfindung zugrundeliegende Aufgabe wird ferner durch die Schaffung eines Verfahrens zur Herstellung des Biokatalysators gelöst, bei dem eine wässrige Lösung der die Matrix bildenden Substanz mit 7 bis 35 Gew.% TiO₂ gemischt, in dieser Mischung 0,1 bis 20 Gew.% biologisch aktives Material dispergiert, die Dispersion bei einem Druck von 800 bis 10 mbar entgast und die entgaste Dispersion aus einer Düse in ein Fällbad getropft wird. Der Biokatalysator erhält insbesondere durch die nach dem erfindungsgemäßen Verfahren vorgesehene Entgasung der Dispersion das gewünschte spezifische Gewicht. Alle Verfahrensschritte werden bei einer Temperatur von 15 bis 40°C durchgeführt, wobei sterile Bedingungen eingehalten werden können. Als Fällbad für die Biokatalysatoren, deren Matrix aus Biopolymeren, wie Alginat, Pectin oder Carragenan, besteht, werden wässrige Lösungen zwei-oder dreiwertiger Kationen, insbesondere Ca²⁺ oder Al³⁺, verwendet, während die chitosanhaltigen Katalysatoren mit einer wässrigen Lösung gefällt werden, die mehrwertige Anionen, insbesondere Polyphosphate, enthält. In weiterer Ausgestaltung der Erfindung ist vorgesehen, daß die Lösung der die Matrix bildenden Substanz 70 bis 99 Gew.% Wasser enthält. Hierdurch wird sichergestellt, daß die TiO₂-Teilchen gleichmäßig eingemischt werden können.

Nach der Erfindung ist es besonders vorteilhaft, wenn die aus der Düse austretenden Tropfen im freien Fall einen 0,2 bis 2 m langen Gasraum passieren, der ein inertes Gas oder Luft enthält, bevor die Tropfen in das Fällbad eintreten, denn durch diese Maßnahme erhalten die aus der Düse austretenden Tropfen eine nahezu kugelförmige Gestalt, die sich auch durch das Auftreffen auf das Fällbad nicht wesentlich verändert. Ferner ist es nach der Erfindung besonders vorteilhaft, wenn in den Gasraum reaktive Dämpfe, wie Acetaldehyd, eingebracht werden, die eine oberflächliche Härtung der Katalysatorperlen bewirken. Im Gasraum können Temperaturen bis 121°C herrschen, da die Katalysatorperlen im Gasraum nur eine kurze Verweilzeit haben und durch eine höhere Temperatur nicht inaktiviert werden. Schließlich ist nach der Erfindung vorgesehen, daß die aus der Düse austretenden Tropfen im Fällbad 0,1 bis 24 Stunden verbleiben. Während dieser Zeit kommt es zu einer Vernetzung zwischen der die Matrix bildenden Substanz und den im Fällbad vorhandenen mehrwertigen Ionen. Durch die Vernetzung erhalten die Tropfen eine feste Struktur. Die harten Katalysatorkugeln sind mehrere Monate lagerfähig und können vorteilhaft in einem Fließbettreaktor verwendet werden. Vor ihrem Einsatz können die Katalysatorkugeln ggf. mit Sauerstoff aktiviert werden.

Der Gegenstand der Erfindung wird nachfolgend anhand eines Ausführungsbeispiels näher erläutert.

1 635 kg doppelt destilliertes Wasser werden mit 3,5 g Penicilin K gemischt, wodurch eine Infektion des Katalysators mit Fremdkeimen für einen langen Zeitraum unterdrückt wird. Anschließend werden in dieser Mischung 300 kg der Hefe saccharomyces cerevisiae dispergiert, wobei die Hefe einen Trockensubstanzgehalt von 30 Gew.% hat. In diesem Gemisch werden dann 600 kg TiO₂ dispergiert, das mit einem Aluminiumsalz behandelt ist und einen Al₂O₃-Gehalt von 2 Gew.% aufweist. In der Suspension wird dann eine Lösung dispergiert, die aus 1 000 kg doppelt destilliertem Wasser, 60 kg Natriumalginat, 360 g Ölsäure, 180 g Ergosterin und 2 l eines Emulgators auf Fettsäurebasis besteht. Die Ölsäure und das Ergosterin wirken während der Lebensdauer des Katalysators insbesondere für seine Ruhephasen als Wachstumssubstrat.

Die Katalysator-Mischung wird anschließend in einem Vakuumgefäß bei 800 bis 10 mbar entgast. Während der Entgasung wird langsam gerührt. Die entgaste Katalysator-Mischung wird mit Preßluft oder Inertgas bei einem Druck von ca. 3 bar in einen Düsenkopf gefördert, der eine Vielzahl von Kappillardüsen aufweist, die jeweils einen Innendurchmesser von 0,8 mm haben. Die Kappillardüsen werden in axialer Richtung von oben nach unten in Austrittsrichtung der Tropfen von Luft oder Inertgas umströmt, wodurch Tropfen definierter Größe abgerissen werden. Die Tropfen fallen durch einen 500 mm langen Gasraum, der Luft enthält, und treffen dann auf das Fällbad auf, welches aus 2 Gew.% CaCl₂ und doppelt destilliertem Wasser besteht. Das Fällbad hat eine Höhe von 1 000 mm, und die Katalysatorperlen erhalten beim Durchgang durch den Gasraum sowie das Fällbad eine runde Form. Die Katalysatorperlen werden am Boden des Fällbades abgezogen und gelangen in eine Behälter, wo sie während 12 Stunden in einer Lösung bewegt werden, die aus 2 Gew.% CaCl₂ und doppelt destilliertem Wasser besteht. Anschließend werden die Katalysatorperlen in Fässer abgefüllt und dort in einer 1 gew.%igen CaCl₂-Lösung bei 4°C gelagert. Die Lagerung kann ohne Aktivitätseinbuße des Biokatalysators mehrere Monate andauern. Die Katalysatormischung erbringt 5 000 l feuchte Katalysatorperlen, die einen Durchmesser von 2,5 mm sowie ein Schüttgewicht von 0,7 kg/l haben.

Der Biokatalysator wird zur Fermentation glukosehaltiger Substrate mit dem Ziel der Äthanolherstellung eingesetzt. Vor dem eigentlichen Beginn der Fermentation wird der Biokatalysator mit einer 5 gew.%igen Glukoselösung unter Zugabe von Luft 5 Tage aktiviert, wobei die Hefezellen in einer dichten Wachstumszone von ca. 100 µm unter der Kugeloberfläche wachsen. Der Biokatalysator hat bei kontinuierlichem Betrieb eine Standzeit von ca. 8 Monaten.

## Patentansprüche

1. Kugelförmiger Biokatalysator, der aus immobilisiertem, biologisch aktivem Material sowie einer ionotropen, gelartigen Matrix aus einem Biopolymer besteht, dadurch gekennzeichnet, daß die Matrix TiO₂ und das biologisch aktive Material homogen verteilt enthält, wobei das TiO₂ Teilchendurchmesser von 0,1 bis 1 µm hat.

2. Biokatalysator nach Anspruch 1, dadurch gekennzeichnet, daß die TiO₂-Teilchen an der Oberfläche mit Al₂O₃ und/oder AlO(OH) dotiert sind.

3. Biokatalysator nach den Ansprüchen 1 bis 2, dadurch gekennzeichnet, daß als biologisch aktives Material Enzyme, Mikroorganismen oder Zellen verwendet werden.

4. Biokatalysator nach den Ansprüchen 1 bis 3, dadurch gekennzeichnet, daß die Matrix aus Biopolymeren, wie Alginat, Pectin, Carragenan oder Chitosan, besteht.

5. Biokatalysator nach den Ansprüchen 1 bis 4, dadurch gekennzeichnet, daß er einen Durchmesser von 0,05 bis 5 mm hat.

6. Verfahren zur Herstellung des Biokatalysators nach den Ansprüchen 1 bis 5, dadurch gekennzeichnet, daß eine wässrige Lösung der die Matrix bildenden Substanz mit 7 bis 35 Gew.% TiO₂ gemischt, in dieser Mischung 0,1 bis 20 Gew.% biologisch aktives Material dispergiert, die Dispersion bei einem Druck von 800 bis 10 mbar entgast und die entgaste Dispersion aus einer Düse in ein Fällbad getropft wird.

7. Verfahren nach Anspruch 6, dadurch gekennzeichnet, daß die Lösung der die Matrix bildenden Substanz 70 bis 99 Gew.% Wasser enthält.

8. Verfahren nach den Ansprüchen 6 bis 7, dadurch gekennzeichnet, daß die aus der Düse austretenden Tropfen im freien Fall einen 0,2 bis 2 m langen Gasraum passieren, der ein inertes Gas oder Luft enthält, bevor die Tropfen in das Fällbad eintreten.

9. Verfahren nach Anspruch 8, dadurch gekennzeichnet, daß in den Gasraum reaktive Dämpfe, wie Acetaldehyd, eingebracht werden.

10. Verfahren nach den Ansprüchen 6 bis 9, dadurch gekennzeichnet, daß die aus der Düse austretenden Tropfen im Fällungsbad 0,5 bis 24 Stunden verbleiben.

## Claims

1. A spherical biocatalyst, which consists of immobilised, biologically active material and also an ionotropic, gel-like matrix of a biopolymer, characterised in that the matrix contains TiO₂ and the biologically active material in a homogenous distribution, the TiO₂ having particle diameters of 0.1 to 1 µm.

2. A biocatalyst according to Claim 1, characterized in that the TiO₂ particles are doped on the surface with Al₂O₃ and/or AlO(OH).

3. A biocatalyst according to Claims 1 and 2, characterised in that enzymes, microorganisms or cells are used as the biologically active material.

4. A biocatalyst according to Claims 1 to 3, characterised in that the matrix consists of biopolymers, such as alginate, pectin, carrageenan or chitosan.

5. A biocatalyst according to Claims 1 to 4, characterised in that it has a diameter of 0.05 to 5 mm.

6. A method for producing the biocatalyst according to Claims 1 to 5, characterised in that an aqueous solution of the substance forming the matrix is mixed with 7 to 35% by weight TiO₂, 0.1 to 20% by weight biologically active material is dispersed in this mixture, the dispersion is degassed at a pressure of 800 to 10 mbar and the degassed dispersion is dropped from a nozzle into a precipitating bath.

7. A method according to Claim 6, characterised in that the solution of the substance forming the matrix contains 70 to 99% by weight water.

8. A method according to Claims 6 and 7, characterised in that the drops emerging from the nozzle pass in free-fall through a 0.2 to 2 m-long gas space which contains an inert gas or air before the drops enter the precipitating bath.

9. A method according to Claim 8, characterised in that reactive vapours, such as acetaldehyde, are introduced into the gas space.

10. A method according to Claims 6 to 9, characterised in that the drops emerging from the nozzle remain in the precipitating bath for 0.5 to 24 hours.

## Revendications

1. Biocatalyseur en forme de bille, qui est constitué d'une matière active biologiquement et immobilisée, ainsi que d'une matrice ionotrope sous forme de gel, en un polymère biologique, caractérisé en ce que la matrice contient, réparti d'une manière homogène du TiO₂ et la matière biologiquement active, TiO₂ ayant un diamètre de particule de 0,1 à 1 µm.

2. Biocatalyseur suivant la revendication 1, caractérisé en ce que les particules de TiO₂ sont dopées à la surface par Al₂O₃ et/ou par AlO(OH).

3. Biocatalyseur suivant la revendication 1 ou 2, caractérisé en ce que comme matières actives biologiquement sont utilisés des enzymes, des microorganismes ou des cellules.

4. Biocatalyseur suivant l'une des revendications 1 à 3, caractérisé en ce que la matrice est en polymère biologique, comme un alginate, une pectine, de la carragénine ou du chitosane.

5. Biocatalyseur suivant l'une des revendications 1 à 4, caractérisé en ce qu'il a un diamètre de 0,05 à 5 mm.

6. Procédé de préparation du biocatalyseur suivant les revendications 1 à 5, caractérisé en ce qu'il consiste à mélanger une solution aqueuse de la substance formant la matrice à 7 à 35 % en poids de TiO₂, à disperser dans ce mélange de 0,1 à 20 % en poids d'une matière active biologiquement, à dégazer la dispersion sous une pression de 800 à 10 mbar et à faite s'égoutter goutte à goutte la dispersion dégazée par une buse dans un bain de précipitation.

7. Procédé suivant la revendication 6, caractérisé en ce que la solution de la substance formant la matrice contient de 70 à 99 % en poids d'eau.

8. Procédé suivant la revendication 6 ou 7, caractérisé en ce qu'il consiste à faire passer les gouttes sortant de la buse en chute libre dans un espace réservé au gaz de 0,2 à 2 m de longueur, qui contient un gaz inerte ou de l'air, avant que les gouttes n'entrent dans le bain de précipitation.

9. Procédé suivant la revendication 8, caractérisé en ce qu'il consiste à introduire, dans l'espace réservé au gaz, des vapeurs réactives, comme de l'acétaldéhyde.

10. Procédé suivant l'une des revendications 6 à 9, caractérisé en ce qu'il consiste à laisser les gouttes sortant de la buse dans le bain de précipitation pendant 0,5 à 24 heures.
